# EUROPÄISCHE PATENTSCHRIFT

(11) **EP 4 009 930 B1**
(45) Veröffentlichungstag und Bekanntmachung des Hinweises auf die Patenterteilung: **26.11.2025**
(21) Anmeldenummer: 20760440.6
(22) Anmeldetag: 19.08.2020
(51) Int. Cl.: A61F 13/505, A61F 13/49, A61F 13/66

(54) **WINDEL MIT TRENNBAREM ABSORPTIONSKÖRPER**
DIAPER WITH SEPARABLE ABSORPTION BODY
COUCHE CULOTTE AVEC UN CORPS ABSORBENT SÉPARABLE

(30) Priorität: 24.09.2019 DE 102019006683
(43) Veröffentlichungstag der Anmeldung: 15.06.2022
(73) Patentinhaber: Gottlieb Binder GmbH & Co. KG, 71088 Holzgerlingen (DE)
(72) Erfinder: POULAKIS, Konstantinos, 71157 Hildrizhausen (DE)
(74) Vertreter: Kohler Schmid Möbus Patentanwälte
(86) Internationale Anmeldenummer: PCT/EP2020/073239
(87) Internationale Veröffentlichungsnummer: WO 2021/058202

(56) Entgegenhaltungen:
- EP-A1- 2 810 629
- EP-A1- 2 810 629
- WO-A1-2016/138466
- WO-A1-2016/138466
- DE-U1- 20 302 153
- DE-U1- 20 302 153
- US-A1- 2005 148 984
- US-A1- 2010 179 496
- US-A1- 2010 179 496
- US-A1- 2012 107 570
- JANIS BEENEN: "Katzenstreu aus Windeln", S�DDEUTSCHE ZEITUNG, 5 January 2019 (2019-01-05), XP055742460, Retrieved from the Internet <URL:https://www.sueddeutsche.de/wirtschaft/windeln-recycling-umweltschutz-1.4274998?print=true> [retrieved on 20201021]
- JANIS BEENEN: "Katzenstreu aus Windeln", SÜDDEUTSCHE ZEITUNG, 5 January 2019 (2019-01-05), XP055742460, Retrieved from the Internet <URL:https://www.sueddeutsche.de/wirtschaft/windeln-recycling-umweltschutz-1.4274998?print=true> [retrieved on 20201021]

## Beschreibung

Die Erfindung betrifft eine Windel, wie eine Baby- oder Inkontinenzwindel, mit den Merkmalen im Oberbegriff von Anspruch 1.

Dahingehende Windeln, wie sie auf dem weltweiten Markt benutzt werden, sind regelmäßig als Einwegwindeln konzipiert, was zu erheblichen Abfallmengen führt, deren umweltfreundliche Entsorgung als problematisch anzusehen ist. Zwar werden heutzutage Einwegwindeln auf dem Markt als sog. Öko-Windeln in umweltfreundlichen Varianten angeboten, die einen höheren Anteil biologisch abbaubarer Bestandteile aufweisen, die zumindest teilweise aus nachhaltigen Ressourcen gewonnen sind; allein werden diese Öko-Windeln nicht über den Biomüll, sondern über den üblicherweise zur Verbrennung in Heizkraftwerken vorgesehenen Restmüll entsorgt, was nicht als umweltfreundlich bezeichnet werden kann.

Sofern (Mehrweg-)Stoffwindeln Verwendung finden, ist deren Ökobilanz gegenüber Einwegwindeln gemäß Inhalt aktueller Studien nicht zwingend besser, da diese für eine erneute Nutzung zu waschen sind.

Zwar ist in der Praxis schon vorgeschlagen worden, benutzte Einwegwindeln weiter zu verwerten. So soll die saugstarke Cellulose als Absorptionskörper nach Trennung von den sonstigen Bestandteilen durch Lösen der insoweit miteinander verklebten Schichten und ihrer Reinigung als Katzenstreu Verwendung finden (Quelle: Süddeutsche.de, Wirtschaft, 5. Januar 2019). Allein auch diese Art des Recyclings mag sich im Hinblick auf das getrennte Einsammeln der benutzten Windeln und deren Transport zu einer zentralen Verwertungs- und Aufbereitungsstelle aus Umweltschutzgründen heraus als problematisch erweisen.

Die WO 2016/138466 A1 beschreibt eine Windel, wie eine Baby- oder Inkontinenzwindel, mit den Merkmalen im Oberbegriff von Anspruch 1, bestehend aus mindestens einer Außenhülle und mit einem mit der Hülle verbundenen Absorptionskörper, wobei mittels eines zwischen Außenhülle und Absorptionskörper wirkenden mechanischen Verschlusssystems der Absorptionskörper von der Hülle separierbar ist.

Weitere Windeln gehen aus der DE 203 02 153 U1, der US 2010/0179496 A1 und der EP 2 810 629 A1 hervor.

US 2012/107570 A1 und US 2005/148984 A1 offenbaren eine Windel und eine Damenbinde, die ein Befestigungssystem aufweisen, wobei das Befestigungssystem mit einem Streifen von geckoartigem Material ausgebildet ist

Ausgehend von diesem Stand der Technik stellt sich daher die Erfindung die Aufgabe, die Ökobilanz von Einwegwindeln der genannten Art zu verbessern.

Eine dahingehende Aufgabe löst eine Windel mit den Merkmalen des Patentanspruchs 1 in seiner Gesamtheit.

Gemäß dem Kennzeichen von Anspruch 1 ist vorgesehen, dass das Verschlusssystem eine Folie mit einer Vielzahl von vorstehenden Anhaftköpfen aufweist, die mittels Adhäsion mit ihren stirnseitigen Kopfflächen an der Außenhülle und/oder dem Absorptionskörper in wieder lösbarer Weise anhaften. Das dahingehende Verschlusssystem vertreibt die Schutzrechtsinhaberin unter ihrer Marke "Gecko" als sog. Gecko-Folie, die aufgrund ihrer dünnschichtigen Ausprägung und ihrer Nachgiebigkeit in allen Richtungen für den jeweiligen Träger einer solchen Windel als ausgesprochen angenehm und komfortabel empfunden wird. Beispielhaft sind derartige Adhäsionslösungen in DE 10 2010 044 660 A1 und DE 10 2010 032 855 A1 der Schutzrechtsinhaberin offenbart.

Dadurch, dass mittels eines zwischen Außenhülle und Absorptionskörper wirkenden mechanischen Verschlusssystems der Absorptionskörper von der Hülle separierbar ist, lässt sich der benutzte Absorptionskörper, insbesondere wenn er aus ökologisch abbaubaren Zellstoffmaterialien aufgebaut ist, von den sonstigen Komponenten, wie der Außenhülle der Windel, mechanisch trennen und im Biomüll entsorgen und die Außenhülle, insbesondere wenn sie aus üblichen verrottbaren Kunststoffmaterialien aufgebaut ist, in den Verwertungskreislauf rückführen, wofür in Deutschland die Abgabe über den "gelben Sack" oder eine sonstige Wiederverwertung im Rahmen des "grünen Punktes" steht. Da eine mechanische Trennung im Vordergrund steht, die zeitnah mit geringen Betätigungskräften, auch durch den Endverbraucher, in hygienisch einwandfreier Weise durchgeführt werden kann, ist das Lösen von Klebstoffverbindungen entbehrlich, wobei die eingesetzten Klebstoffmittel auch aus Gründen des Umweltschützens heraus problematischer einzustufen sind als die hier propagierte mechanische Verbindungslösung.

Erfindungsgemäß ist vorgesehen, dass der Absorptionskörper der Windel einen Saugkörper aufweist, insbesondere in Form von Zellstoffflocken, die in einer fluiddurchlässigen, geschlossenen Absorberhülle, insbesondere aus Vliesmaterial, aufgenommen sind, das besonders bevorzugt biologisch abbaubar ist.

Bei einer anderen nicht-erfindungsgemäßen Ausführungsform einer Windel ist vorgesehen, dass das genannte Verschlusssystem einen Klettenhaftverschluss aufweist, bestehend aus Haken und/oder Pilzköpfen auf der einen Seite und mit diesen in wieder lösbarer Weise verhakbaren Schlaufen oder Fasern oder weiteren Pilzköpfen auf der anderen Seite. Ein dahingehendes Verschlusssystem nebst einem Verfahren zum Herstellen desselben ist aus DE 196 46 318 A1 der Schutzrechtsinhaberin bekannt. Verschlusssysteme, bei denen auf beiden Seiten des Verschlussmaterials Pilzköpfe in wieder lösbarer Weise miteinander verrastet werden, bezeichnet man fachsprachlich auch als hermaphroditische Verschlüsse.

Insbesondere ist dann in vorteilhafter Weise für die Windel ferner vorgesehen, dass die Schlaufen oder Fasern des einen Teils des Klettenhaftverschlusses aus mindestens einem Gewirk, Gestrick, Gewebe, Gaze, Flausch, Vlies oder Velours gebildet sind. Diesbezügliche Grundgewebe aus Kett- und Schussfäden aufgebaut und mit auf der Vorderseite des Grundgewebes vorstehenden Funktionselementen in Form von Schlaufen sind beispielhaft für ein schwer entflammbares Verschlussteil in DE 10 2013 022 112 A1 der Schutzrechtsinhaberin beschrieben.

Die Absorberhülle ist über ihre gesamte Fläche, die der Innenseite der Außenhülle zugewandt ist, mit der einen Seite mit den Haken oder Pilzköpfen des Klettenhaftverschlusses in wieder lösbarer Weise verhakt.

Alternativ besteht die Möglichkeit, vergleichbar die Absorberhülle mit der Gecko-Folie auszugestalten.

Bei einer Ausführungsform der Windel ist vorgesehen, dass die Außenhülle aus einem folienartigen Verschlussmaterial (Gecko-Folie) gebildet ist, dessen Haken oder Pilzköpfe dem Absorberkörper zugewandt sind. Dergestalt lässt sich die Außenhülle der Windel mit nur einer Lage der Folie gestalten, was den Tragekomfort erhöht und die Entsorgung bei verbesserter Ökobilanz ermöglicht.

Vorzugsweise ist hierbei vorgesehen, für eine Vereinzelung der Haken oder Pilzköpfe der Klettverschlussfolie als der Außenhülle diese unidirektional und im Bedarfsfall auch bidirektional zu recken, was das Folienmaterial dünner werden lässt; gleichzeitig bleibt aber die Reißfestigkeit erhalten, was wiederum die zu entsorgende oder zu verwertende Restmenge der Windel nach Entfernen des Absorptionskörpers verringert, und durch die Dünnheit der Folie mit der Vereinzelung der Verschlusselemente ist wiederum der Tragekomfort für den Nutzer erhöht.

Im Folgenden wird die Windel anhand zweier Ausführungsbeispiele näher erläutert. Dabei zeigen in prinzipieller und nicht maßstäblicher Darstellung die
- Fig. 1: eine Draufsicht auf ein erstes Ausführungsbeispiel der Windel;
- Fig. 2: eine der Fig. 1 entsprechende Darstellung eines zweiten Ausführungsbeispiels der Windel; und
- Fig. 3 und 4: zwei voneinander verschiedene Verschlusssystemteile für einen Absorptionskörper, wie er für eine Windel nach den Fig. 1 und 2 verwendbar ist.

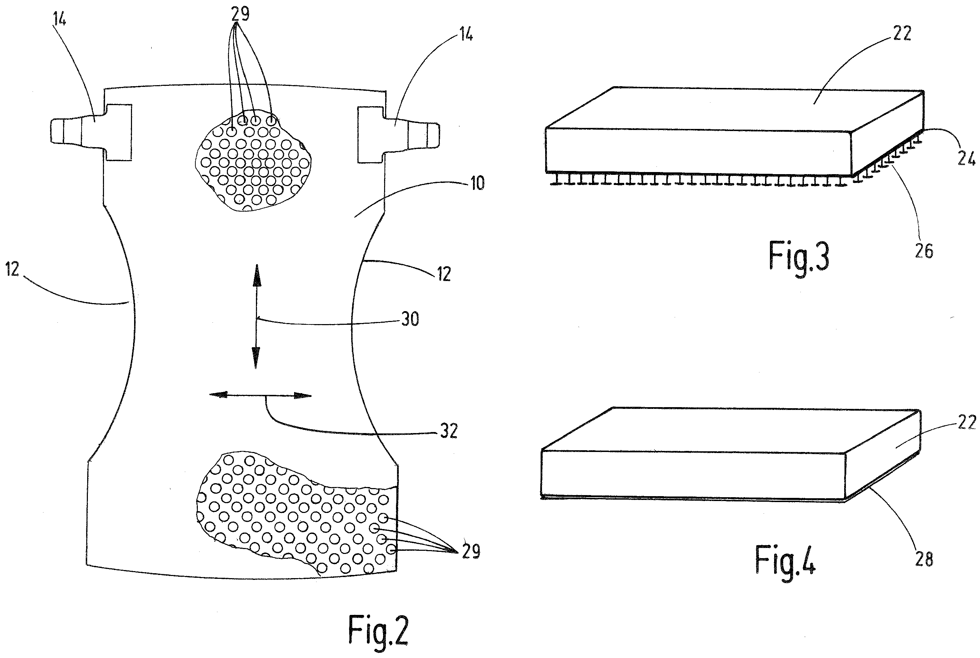

Die Ausführungsformen der Fig. 2 und 3 dienen lediglich der Erläuterung des Hintergrundes der Erfindung und sind nicht Gegenstand eines Anspruches.

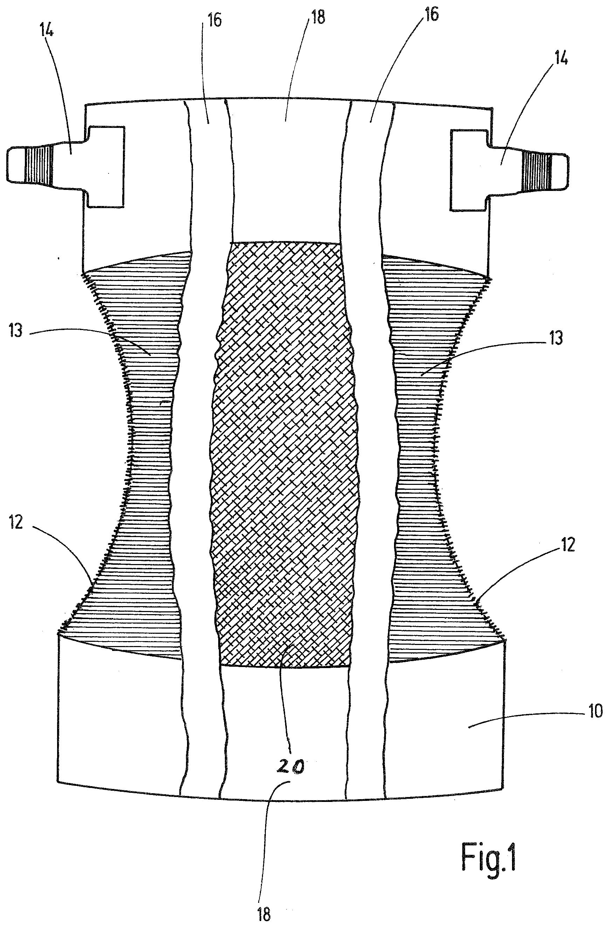

Die Fig. 1 zeigt in ebener Abwicklung die Außenhülle 10 einer Einwegwindel in Form einer Baby- oder Inkontinenzwindel für Erwachsene. Die Außenhülle 10 ist entlang ihrer beiden einander gegenüberliegenden Längsränder 12 tailliert ausgebildet, so dass insoweit Beinöffnungen für einen Windelnutzer gebildet sind. Die eine Art Bündchen ausbildenden beiden Längsbereiche 13 sind aus einem bei Windeln üblichen elastischen Material gebildet, was den Tragekomfort für den Nutzer erhöht. An ihrem in Blickrichtung auf die Fig. 1 gesehen oberen Ende der Außenhülle 10 sind in üblicher Weise zwei Verschlüsse 14 angebracht, mit denen die Windel an einem Nutzer festgelegt, und nach Gebrauch aufgerollt, verschlossen werden kann. Hierfür weisen die beiden Verschlüsse in Richtung ihrer beiden freien Enden hin einen Klebstoff oder Klettenhaftverschlussteile (beides nicht dargestellt) auf, mit dem die Verschlüsse 14 auf der Vorderseite der Windel in einem sog. Targetbereich das dauerhafte Anlegen der Windel ermöglichen.

Entlang der beiden Längsränder 12 verlaufen in Blickrichtung auf die Fig. 1 gesehen rechts und links von der Mitte der Hülle 10 und von oben nach unten durchgehend zwei Abdeckstreifen 16, die zwischen sich unter Freilassen von zwei Endbereichen 18 einen Teil 20 eines Klettenhaftverschlusses aufnehmen. Die Abdeckstreifen 16 sind in Richtung zu den beiden Längsrändern 12 fest mit der Hülle 10 verbunden und lassen sich taschenartig in Richtung ihrer beiden benachbarten Randbereiche abheben, um dergestalt einen Absorptionskörper 22, randseitig übergreifend und insoweit abdeckend, aufnehmen zu können. Der Teil 20 ist im vorliegenden Ausführungsbeispiel aus einem Schlaufenmaterial gebildet, das als durchgehender Streifen unter die beiden einander zugewandten freien Enden der Abdeckstreifen 16 eingefügt und mit der Außenhülle 10 fest verbunden, beispielsweise fest verklebt, ist. Anstelle eines eigenständigen Schlaufenmaterials kann aber auch unmittelbar ein Fasermaterial der Außenhülle 10 für den noch zu beschreibenden Verhakungsvorgang vorgesehen sein.

Ein geeignetes Absorbermaterial ist beispielhaft in der Fig. 3 in Form des Absorptionskörpers 22 dargestellt, der eine fluiddurchlässige Gazehülle aufweist, die Zellstoffflocken im Inneren des Absorptionskörpers 22 umfasst, die der Aufnahme von Fluid dienen, regelmäßig gebildet aus den Ausscheidungen des Windelnutzers.

Auf der Unterseite des streifen- oder blockförmig ausgebildeten Absorptionskörpers 22 ist flächig auf einem Trägerband 24 ein in Blickrichtung auf die Fig. 3 gesehen nach unten vorstehendes Verschlussmaterial in Form von Haken 26 vorgesehen. Wird der derart ausgestattete Absorptionskörper 22 in die ebene Abwicklung der Hülle 10 nach der Fig. 1 aufgesetzt, verhaken die Haken 26 auf der einen Seite des propagierten Klettenhaftverschlusses mit den Schlaufen auf der anderen Seite des Klettenhaftverschlusses und bilden insoweit einen wieder lösbaren mechanischen Verschluss aus. Im handelbaren Verkaufszustand wird die Windel mit dem derart über das mechanische Verschlusssystem verbundenen Absorptionskörper 22 ausgeliefert und ist dergestalt gebrauchsfähig. Nach Anlegen der Windel und deren späterem Gebrauch ist der Absorptionskörper 22 entsprechend verschmutzt und kann über das mechanische Verschlusssystem, bestehend aus Haken 26 und Schlaufen des Teils 20, von der sonstigen Außenhülle 10 der Windel getrennt werden. Der Absorptionskörper 22, der aus biologisch abbaubaren Materialien besteht, lässt sich dann getrennt von der Außenhülle 10, beispielsweise im Rahmen des Biomülls, entsorgen. Die Außenhülle 10 kann dann wiederum in einem getrennt hiervon verlaufenden Versorgungskreislauf zwecks Erhalts wiederverwendbarer Rohstoffe entsorgt werden. Auch wäre es denkbar, beispielsweise im Rahmen von Kliniken oder Pflegeeinrichtungen, die Außenhülle 10 nur einmal auszuliefern zusammen im Paket mit einer Vielzahl von Absorptionskörpern 22, die dann bei Benutzung gegen neue Körper 22 ausgetauscht werden können bei Erhalt der einzelnen Außenhülle 10 für eine gewisse vorgebbare Nutzungsdauer.

Sofern man als Befestigungszone direkt das Velours- respektive Fasermaterial einer Windel in Form der Außenhülle 10 einsetzt, kann ein separater Teil 20 des Klettenhaftverschlusses in Form des Schlaufenmaterials entfallen, so dass auch keine Klebstoffverbindung zwischen der Außenhülle 10 und dem dahingehenden eigenständigen Teil 20 des Klettenhaftverschlusses vorgesehen werden muss, was wiederum die Herstellkosten reduzieren hilft und der umweltfreundlichen Gestaltung der Windel Vorschub leistet.

Wie bereits dargelegt, haben die als Einwegwindel, Wegwerfwindel oder Höschenwindel bezeichneten Hilfen die vorgestellte Außenhülle 10, die regelmäßig aus Polyethylenmaterialien gebildet ist und einen Saugkörper aufweist. Neuere Entwicklungen erlauben es, dahingehendes Polyethylen aus Zuckerrohr zumindest teilweise mit aufzubauen, das als nachwachsender Rohstoff und mithin biobasiertes Fertigungsmaterial als sehr umweltfreundlich einzustufen ist. Die Windel kann im Bedarfsfall auch aus biobasiertem Polymerwerkstoff oder Polypropylen aufgebaut werden.

Der Saug- oder Absorptionskörper 22 besteht, wie bereits dargelegt, aus Zellstoffmaterialien, insbesondere in Flockenform, die bei modernen Windeln oft mit einem zusätzlichen Superabsorber sowie spezifischen Polymersalzen angereichert sind, um dergestalt hohe Flüssigkeitsmengen im Volumen des Sauganteils des Absorptionskörpers 22 zu binden. Insbesondere wird unter dem Tragedruck die einmal aufgenommene Flüssigkeit nicht, wie bei einem Schwamm, wieder abgegeben. An der Innen- oder Außenschicht der Hülle 10 sind die Klebe- oder Klettlaschen als Verschlüsse 14 vorhanden, mit denen die Windel anlegbar und insbesondere an den Bauchumfang des Nutzers angepasst werden kann. Sie können mit einer Kunststofffolie überzogen sein, um das Durchsickern von Flüssigkeiten aus der Windel zu verhindern. Weitere Bestandteile der Windel können Vaseline, Stearylalkohol, dünnflüssige Paraffine und Extrakte der Aloe barbadensisis sein. Sofern hier naturnahe Materialien zum Einsatz kommen, beeinträchtigen solche Zusatzmaterialien und Zusatzstoffe das umweltfreundliche Recycling und Wiederverwerten der Windel nicht.

Die Haken 26 des Absorptionskörpers 22 können durch Schlaufen und das Schlaufenband als dem Teil 20 der Außenhülle 10 durch ein entsprechendes Hakenverschlussmaterial ersetzt werden. Neben dem genannten Haken-Schlaufen-Verschlusssystem können anstelle der Haken 26 auch in üblicher Weise Pilzköpfe als dem einen Verschlussteil treten, die sich mit einem entsprechenden Pilzkopfmaterial auf Seiten der Außenhülle 10 unter Bildung sogenannter hermaphroditischer Verschlüsse in wieder lösbarer Weise verbinden lassen. Insbesondere wenn der Absorptionskörper 22 direkt mit dem Faser- oder Flauschmaterial der Außenhülle 10 verbunden werden soll, empfiehlt sich erfindungsgemäß der Einsatz der sogenannten Gecko-Folie 28, wie sie beispielhaft in der Fig. 4 dargestellt ist. Die genannte Gecko-Folie 28 weist auf ihrer Unterseite Verschlusselemente in Form von Pilzköpfen auf, die, in Mikroreplizier-Technik hergestellt, von ihren geometrischen Abmessungen her derart klein bemessen sind, dass sie mit bloßem Auge auf ihrer Trägerfolie kaum erkennbar sind. Die freien Stirnseiten der Mikropilzköpfe ermöglichen ein Anhaften an der Außenhülle 10 der Windel überwiegend durch Aufbringen von Adhäsionskräften zwischen der Außenhülle 10 und der Gecko-Folie 28. Besteht der Absorptionskörper 22 aus einem flächigen Absorbermaterial, kann gegebenenfalls die Zellstoffhülle für den Körper 22 auch weggelassen werden und das jeweils vorgesehene Klettenhaftverschlussteil kann dann auch unmittelbar mit dem Absorberkörper verbunden dessen Umhüllung bilden, wobei dann das jeweilige Verschlussteil durch Einbringen einer Perforation fluiddurchlässig zu gestalten ist. Bei dahingehenden Ausgestaltungen wäre es auch denkbar, das Trägerband 24 gemäß der Darstellung nach der Fig. 3 auf beiden Seiten mit einem entsprechenden Verschlussmaterial, Haken oder Pilzköpfe, zu versehen, so dass die eine Seite des Verschlussmaterials dem Verbinden mit dem Absorbermaterial dient und die andere freie Seite für das Verbinden mit der Windelaußenhülle 10 respektive dem Teil 20 zur Verfügung steht.

Die Außenhülle 10 bei der geänderten Ausführungsform nach der Fig. 2 besteht im Wesentlichen aus einem ebenen Folienzuschnitt aus Kunststoffmaterial. Auf der dem Betrachter zugewandten Innenseite der Außenhülle 10 ist durchgehend ein Verschlussmaterial in Form von Pilzköpfen 29 angeordnet, die in vorgebbaren Spalten und Reihen zueinander angeordnet sind. Um den Tragekomfort zu erhöhen, respektive die Entsorgungsmenge klein zu halten, ist die Außenhülle 10 in Längsrichtung gesehen, wie durch die Pfeile 30 angeordnet, unidirektional gereckt worden. Es besteht aber auch die Möglichkeit, im Bedarfsfall quer zur Verlaufsrichtung der Doppelpfeile 30, also gemäß der Pfeildarstellung 32, zu recken oder das Folienmaterial der Außenhülle 10 sowohl in Längsrichtung 30 als auch quer dazu 32 bidirektional zu recken oder zu dehnen. Hierfür vorgesehene Reck- oder Dehnverfahren sind beispielhaft in US 4 001 366 offenbart sowie in DE 694 21 025 025 T2.

Bei der dahingehenden Ausgestaltung wird dann das faserige Absorbermaterial des Absorptionskörpers 22 direkt mit der Außenhülle 10 auf deren Innenseite verbunden. Ferner besteht die Möglichkeit, den in Fig. 3 gezeigten Absorptionskörper 22 einzusetzen mit der Maßgabe, dass das Trägerband 24 auf seiner Unterseite dann kein Hakenverschlussmaterial 26 aufweist, sondern vielmehr schlaufenförmige Verbindungskörper (nicht dargestellt) als Teil des Klettenhaftverschlusssystems. Auch kann anstelle von Haken als Verschlusselemente für Schlaufen die Pilzkopfgestaltung treten. In Anbetracht dessen, dass weltweit jährlich mehrere Milliarden Wegwerfwindeln zu entsorgen sind, kommt der vorliegenden Erfindung im Hinblick auf eine sinnfällige Abfallvermeidung eine hohe Bedeutung zu. Der Absorptionskörper kann gleichfalls aus kompostierbaren Materialien aufgebaut sein.

## Patentansprüche

1. Windel, wie eine Baby- oder Inkontinenzwindel, bestehend aus mindestens einer Außenhülle (10) und mit einem mit der Hülle (10) verbundenen Absorptionskörper (22), wobei mittels eines zwischen Außenhülle **(10)** und Absorptionskörper (22) wirkenden mechanischen Verschlusssystems der Absorptionskörper (22) von der Hülle (10) separierbar ist, **dadurch gekennzeichnet, dass** das Verschlusssystem eine Folie (28) mit einer Vielzahl von vorstehenden Anhaftköpfen aufweist, die mittels Adhäsion mit ihren stirnseitigen Kopfflächen an der Außenhülle (10) und/oder dem Absorptionskörper (22) in wieder lösbarer Weise anhaften,
wobei der Absorptionskörper (22) einen Saugkörper aufweist, insbesondere in Form von Zellstoffflocken, die in einer fluiddurchlässigen, geschlossenen Absorberhülle, insbesondere aus Vliesmaterial, aufgenommen sind,
wobei die Absorberhülle über ihre gesamte Fläche, die der Innenseite der Außenhülle zugewandt ist, mit der Folie (28) ausgestaltet ist.

## Claims

1. Diaper, such as a baby diaper or incontinence diaper, consisting of at least one outer shell (10) and having an absorbent body (22) connected to the shell (10), wherein the absorbent body (22) can be separated from the shell (10) by means of a mechanical closure system acting between the outer shell (10) and the absorbent body (22), **characterized in that** the closure system comprises a film (28) with a plurality of protruding adhesive heads, the front-side head surfaces of which are attached by means of adhesion to the outer shell (10) and/or the absorbent body (22) in a releasable manner,
wherein the absorption body (22) comprises a suction body, in particular in the form of cellulose flakes, which are accommodated in a fluid-permeable, closed absorber shell, in particular made of nonwoven material,
wherein the absorber shell is designed with the film (28) over its entire surface facing the inside of the outer shell.

## Revendications

1. Couche-culotte du type couche-culotte pour bébés ou pour incontinents, constituée d'au moins une enveloppe extérieure (10) et d'un corps absorbant (22) relié à ladite enveloppe (10), lequel corps absorbant (22) peut être dissocié d'avec ladite enveloppe (10) au moyen d'un système mécanique de fermeture agissant entre l'enveloppe extérieure (10) et le corps absorbant (22), **caractérisée par le fait que** le système de fermeture est muni d'un film (28) doté d'une multiplicité de têtes adhérentes en saillie qui demeurent attachées par adhérence à l'enveloppe extérieure (10) et/ou au corps absorbant (22), de manière libérable, par leurs faces frontales extrêmes,
ledit corps absorbant (22) comportant un corps d'aspiration qui revêt, en particulier, la forme de flocons de cellulose logés dans une enveloppe absorbante fermée, perméable aux fluides, consistant notamment en un matériau non tissé,
laquelle enveloppe absorbante est pourvue du film (28) sur l'intégralité de sa surface pointant vers la face intérieure de l'enveloppe extérieure.
